# EUROPEAN PATENT APPLICATION

(11) **EP 4 613 239 A1**
(43) Date of publication of application: **10.09.2025**
(21) Application number: 24305348.5
(22) Date of filing: 07.03.2024
(51) Int. Cl.: A61F 2/00

(54) **IMPLANTABLE-MESH INTRODUCER INCLUDING A CAPE AND METHODS THEREOF**

(71) Applicant: Sofradim Production, 01600 Trevoux (FR)
(72) Inventor: BAILLY, Pierre, 01600 TREVOUX (FR); BRUNE, Thierry, 01600 TREVOUX (FR)
(74) Representative: Germain Maureau

(57) **Abstract**

A mesh introducer comprising a push stick extending between a proximal end portion and a distal end portion; a cape extending between a free proximal end portion and a fixed distal end portion, the fixed distal end portion of the cape secured to the distal end portion of the push stick.

## Description

### Technical Field

The present disclosure describes an implantable-mesh introducer, and particularly an implantable-mesh introducer including a cape configured to cover the mesh during introduction into a patient and release the mesh inside the patient.

### Background

In laparoscopic and/or robotic surgery, one or more trocars may be required to allow passage of surgical tools, such as surgical graspers, scissors, fasteners, lights, and/or optical systems therethrough, as well as to maintain intra-abdominal pressure, e.g., insufflation, in a patient's abdomen to ensure workspace for the surgery (pneumoperitoneum).

Trocars may have different diameters to accommodate the passage of such surgical tools, as well as implants also having variable diameters or sizes. Generally, trocars commonly used may be 5-15 mm diameter trocars for laparoscopic or robotic surgery. Particularly, 5, 10, 12, or 15 mm diameter trocars may be used in traditional laparoscopy and 8 and 12 mm diameter trocars may be used in robotic surgery.

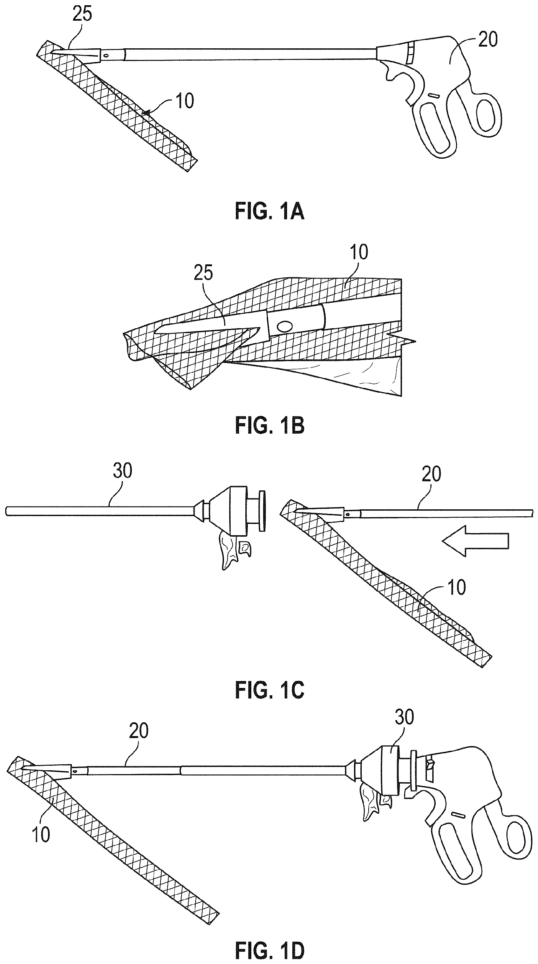

As shown in Figs. 1A-1D, for some surgeries, it may be necessary to introduce an implantable surgical mesh 10 through a trocar 30 via a surgical tool 20, such as a surgical grasper. As best shown particularly in Fig. 1A, the surgical tool 20 may include a tool handle having a surgical arm extending therefrom and including a pair of jaw members 25 extending therefrom. The jaw members 25 may be used to directly pinch a distal end portion of the implantable-mesh 10 for introduction into a patient. As further shown in Figs. 1C and 1D, the pinching of the mesh 10 by the jaw members 25 is maintained throughout insertion into a patient. Essentially, a portion of the surgical tool 20 is passed through the trocar 30 with the jaw members 25 tightly pinched against the mesh 10 and dragging the mesh 10 through the trocar 30 under pressure. The combination of pinching and dragging of the mesh 10 by the tool 20 can damage the mesh 10, such as by cutting, stretching, or denting the mesh 10. In addition, since the mesh 10 is uncovered prior to and during insertion, the mesh 10 can be easily contaminated any time prior to complete insertion.

Also, the external diameter of the tool 20, e.g., a surgical grasper, has to be smaller than the internal diameter of the trocar 30 to be able to leave room for the rolled or folded mesh 10 for both the mesh 10 and the tool 20 to pass through the trocar 30. For example, a surgical tool 20 including a 5 mm external diameter may be used to introduce a mesh 10 having less than about a 5mm diameter when using a 10 mm diameter trocar.

Difficulty can arise when trying to pass a mesh through a trocar which is too small for the mesh alone or with the surgical tool. Since the mesh size is dependent on the size of the hernia defect and thus not easily reduceable in size, the alternative is typically to replace the trocar with a larger trocar which takes time, increases cost, and produces a larger hole in the patient's abdominal wall which is associated to an increased risk of developing an incisional hernia later on.

It is an object of the present disclosure to provide an implantable-mesh introducer of reduced size to provide more room or volume inside the trocar for the mesh and avoid having to change a trocar based on size.

It is another object of the present disclosure to provide an implantable-mesh introducer configured to cover and protect the mesh from damage and/or contamination prior to insertion.

It is still another object of the present disclosure to provide an implantable-mesh introducer configured to insert a mesh without mechanically fixing (e.g., grasping, pinching, stitching, clipping, tying, adhering, etc.) the mesh to any portion of the introducer thereby reducing the likelihood of stressing and/or damaging the mesh prior to insertion.

### SUMMARY

Implantable-mesh introducers are described herein. The introducers include at least a cape configured to surround an implantable-mesh and at least one cape connector or push stick. The cape is intended to surround at least a majority, if not an entirety, of a mesh during insertion of the mesh into a patient.

In some embodiments, an implantable-mesh introducer includes a push stick and a cape. The push stick extends between a proximal end portion and a distal end portion. The cape extends between a free proximal end portion and a fixed distal end portion. The fixed distal end portion of the cape being secured to the distal end portion of the push stick.

In some embodiments, the push stick is a rigid metal push stick and the cape is a flexible polymeric cape.

In some embodiments, the push stick includes a handle on the proximal end portion of the push stick and a cape connector on a distal end portion thereof. The handle may include a shaped body including one or more rings. The cape connector may include a shaped body including a distal lumen configured to receive and secure a distal end portion of the cape therein.

In some embodiments, the distal end portion of the cape forms a bulbous part configured to be secured within the distal lumen of the cape connector. A cape cord may extend distally from the bulbous part out of the distal lumen of the cape connector before the cape turns around the cape connector extending proximally towards the handle. The cape creates a cape pocket proximal the connector, the cape pocket is configured to receive the compacted mesh therein.

In some embodiments, the cape connector also includes a crimping ring configured to snap into the distal lumen of the connector distal the bulbous part of the cape to maintain the bulbous part of the cape within the distal lumen of the cape connector. The crimping ring may include a longitudinal aperture therethrough. The aperture being configured to receive the cape cord therethrough. In some embodiments, the crimping ring further includes one or more locking tabs configured to lock the cape, and particularly the bulbous part and/or the cape cord, inside the distal lumen of the cape connector.

In some embodiments, the mesh introducer includes a cape and cape connector.

In some embodiments, the mesh introducer includes a cape and push stick.

In some embodiments, the mesh introducer includes a cape, a push stick, and cape connector.

In some embodiments, the mesh introducer includes a cape, a push stick, a handle for the stick, and cape connector.

The cape (or introducer) as described herein is configured to transition from an expanded configuration, prior to insertion into a patient, to a delivery configuration, after insertion into a patient. In the expanded configuration, the cape forms a cape pocket designed to receive and maintain a mesh, and particularly a compacted (e.g., rolled or folded) mesh, therein. In the delivery configuration, the cape pocket is opened or inverted allowing the mesh to freely exit the cape or introducer.

In some embodiments, prior to insertion, the cape (or introducer) can transition from the expanded configuration to a wrapped configuration. In some embodiments, after insertion, the cape (or introducer) can transition from a wrapped configuration to an unwrapped configuration.

Methods of delivering an implantable-mesh into a patient are also provided. The methods may include placing a compacted implantable-mesh inside a cape of an implantable-mesh introducer, pushing the implantable-mesh introducer including the compacted implantable-mesh placed inside the cape through a trocar into a patient until the cape and the mesh are completely through the trocar, and releasing the compacted implantable-mesh from inside the cape into the patient.

Methods of repairing a soft tissue defect in a patient are also provided. The methods may include inserting a mesh in a compacted configuration into a cavity of a patient via a mesh introducer including a cape surrounding the mesh, opening of the cape inside the patient freeing the mesh of the introducer, deploying the mesh into a non-compacted configuration, fixating the mesh in or around the soft tissue defect, and withdrawing the mesh introducer including the cape from the cavity of the patient.

Surgical kits are also provided. The surgical kits may include at least one mesh introducer as described herein. In some embodiments, the surgical kits may include a plurality of mesh introducers of varying sizes to fit varying mesh sizes. In some embodiments, the surgical kits may further include at least one of an implantable-mesh, a surgical grasper, or a trocar.

### BRIEF DESCRIPTION OF THE DRAWINGS

Various embodiments of the kits and/or components are described herein with reference to the drawings wherein:
Figs. 1A-1D are side views of a surgical tool and mesh of the prior art;
Fig. 2A is a schematic side view of an implantable-mesh introducer including a cape as described in at least one embodiment herein;
Fig. 2B is a cross-sectional view of the implantable-mesh introducer of Fig. 2A as described in at least one embodiment herein;
Figs. 2C-2D are schematic side views of the implantable-mesh introducer of Fig. 2A as described in at least one embodiment herein;
Fig. 3A is a side view of an implantable-mesh introducer including a cape as described in at least one embodiment herein;
Fig. 3B is a perspective view of a handle of the implantable-mesh introducer depicted in Fig. 3A as described in at least one embodiment herein;
Fig. 3C is a cross-sectional view of the handle depicted in Fig. 3B as described in at least one embodiment herein;
Fig. 3D is a perspective view of a cape connector of the implantable-mesh introducer depicted in Fig. 3A as described in at least one embodiment herein;
Fig. 3E is a cross-sectional view of the cape connector depicted in Fig. 3D as described in at least one embodiment herein;
Fig. 4A is a side view of an implantable-mesh introducer including a cape as described in at least one embodiment herein;
Fig. 4B is a side view of the implantable-mesh introducer of Fig. 4A as described in at least one embodiment herein;
Fig. 4C is a cross-sectional view of the mesh introducer and mesh of Fig. 4B as described in at least one embodiment herein;
Fig. 5 is a side view of an implantable-mesh introducer including a cape as described in at least one embodiment herein;
Figs. 6A-6F are side views of a method of using a mesh introducer including a cape as described in at least one embodiment herein; and
Figs. 7 and 8 are side views of a mesh introducer including a cape as described in at least one embodiment herein;
Fig. 9A is a schematic side view of an implantable-mesh introducer including a cape as described in at least one embodiment herein;
Fig. 9B is a cross-sectional view of the implantable-mesh introducer of Fig. 9A as described in at least one embodiment herein;
Fig. 9C is a cross-sectional view of a cape connector of the implantable-mesh introducer of Fig. 9A as described in at least one embodiment herein;
Fig. 10 is a perspective side view of a cape connector of an implantable-mesh introducer as described in at least one embodiment herein;
Figs. 11 and 12 are each a cross-sectional view of a cape connector of an implantable-mesh introducer as described in at least one embodiment herein;
Fig. 13A is a schematic perspective view of a cape connector of an implantable-mesh introducer as described in at least one embodiment herein;
Fig. 13B is a cross-sectional view of the cape connector of Fig. 13A as described in at least one embodiment herein;
Fig. 13C is a bottom view of the cape connector of Fig. 13A as described in at least one embodiment herein;
Figs. 14A and 14B are a perspective view and side view, respectfully, of a cape connector as described in at least one embodiment herein;
Fig. 15A is a perspective and side view of a retainer ring as described in at least one embodiment herein; and
Figs. 15B-15D are side views of a method of using a mesh introducer including a cape and at least one retainer ring as described in at least one embodiment herein.

### DETAILED DESCRIPTION

The present disclosure describes implantable-mesh introducers and surgical kits including at least one implantable-mesh introducer. The mesh introducer includes at least a cape configured to wrap around an implantable-mesh, and particularly an implantable-mesh in a compacted configuration, i.e., a folded and/or rolled implantable-mesh.

The cape as described herein is a thin sheet of polymeric material sufficient for wrapping and/or unwrapping around an implantable-mesh for delivery into a patient's body. Suitable cape materials display a coefficient of friction sufficient, e.g., low enough, for limiting the forces between the trocar and the introducer reducing stress to the mesh during insertion, as well as provide a high tensile strength allowing the cape to be very thin thereby reducing the volume inside the trocar provided by introducer. Some non-limiting examples of suitable polymeric materials include, but are not intended to be limited to, high-density polyethylene, ultra-high molecular weight polyethylene, polyether ether ketone (PEEK), polyamides, polytetrafluoroethylene (PTFE), polyethylene terephthalate (PET), polypropylene, and combinations thereof. In some embodiments, the cape is made of high-density polyethylene.

The cape may have a thickness less than or equal to about 100 microns. In some embodiments, the thickness of the cape may range from about 0.1 to about 50 microns. In some embodiments, the thickness of the cape may range from about 1 to about 25 microns. In some embodiments, the thickness of the cape may range from about 5 to about 15 microns. In some embodiments, the thickness of the cape may be about 10 microns.

In addition to being thin, the cape is designed to display a lower coefficient of friction when slid across the inside of the trocar than the mesh being slid directly across the inside of the trocar without the cape at least partially positioned therebetween.

The introducer may also include a push stick to which the cape is temporarily or permanently connected thereto. The cape may also be configured to wrap around at least a portion, if not a majority or all, of the push stick.

The push stick can be made of any material suitable for passing the cape, alone or with a surgical mesh wrapped therein, into or out of a patient's body via trocar or opening in a patient's body. Some non-limiting examples of suitable materials used to form the push stick include, but are not intended to be limited to, metal such as stainless steel, silver, gold, titanium, aluminum, and the like, and/or a polymeric material such as a polycarbonate, polyoxymethylene, polypropylene, polyethylene terephthalate glycol, and the like, as well as combinations thereof. In some embodiments, the push stick is a rigid push stick made of metal. The metal material is sufficiently rigid to provide a good pushing force into a patient without buckling or failing, while maintaining a thickness or diameter as small as possible reducing volume of an introducer inside the trocar. In some embodiments, the metal rigid push stick is made of stainless steel.

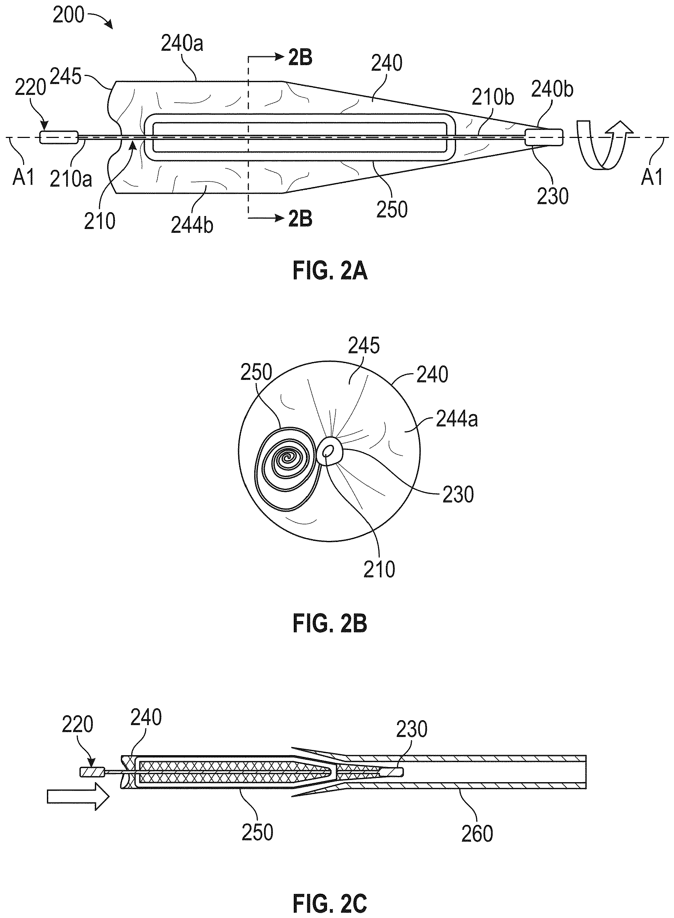

Turning to Figs. 2A-2D, one or more embodiments are depicted of an implantable-mesh introducer 200 as described herein, as well as one or more methods of introducing an implantable-mesh 250, via the introducer 200, into a patient through a trocar 260. The depicted introducer 200 includes at least a push stick 210 and a cape 240 secured thereto. The push stick 210 defines a longitudinal axis A₁ extending between a proximal end portion 210a and distal end portion 210b. The proximal end portion 210a of the stick 210 may include a handle 220. The distal end portion 210b of the stick 210 may include a cape connector 230.

The cape 240 extends between a proximal end portion 240a and a distal end portion 240b. The distal end portion 240b of the cape 240 is secured to the distal end portion 210b of the stick 210 by the cape connector 230. The proximal end portion 240a of the cape 240 is free of and/or unsecured to the push stick 210. The cape 240 extends proximally from the cape connector 230 along a portion of the longitudinal axis A₁ of the stick 210 defining a pocket 245 configured to receive the implantable-mesh 250 therein. The mesh 250 is received in the pocket 245 in a compacted form, i.e., folded, rolled, etc. In some embodiments, at least a portion (and/or a majority) of a longitudinal length of the push stick 210 is also be received within the pocket 245 (Fig. 2B), however the mesh 250 remains free of the push stick 210, i.e., the mesh 250 is not rolled or folded directly around the push stick 210. Rather, the folded or rolled mesh 250 is compacted prior to being combined with the introducer 200 and is secured inside the pocket 245 between the push stick 210 and the cape 240.

In some embodiments, the cape connector 230 permanently secures the cape 240 to the push stick 210. Some non-limiting examples of permanent cape connectors include one or more of glues, adhesives, rivets, pins, screws, welding, and the like.

After inserting the implantable surgical mesh 250 into the cape 240 (and/or pocket 245), the cape 240 can be wrapped around the mesh 250. For example, as indicated by the arrow of Fig. 2A, the stick 210 can be rotated, particularly via the handle 220, in a clockwise (or counter- clockwise) direction to wrap the cape 240 around the mesh 250 (and/or the stick 210) inside the pocket 245, as further depicted in Fig. 2B, prior to insertion.

As depicted in Fig. 2C, the cape 240 and the mesh 250, in the wrapped configuration, can be moved distally (as indicated by the arrow of Fig. 2C) through a trocar 260 into a patient, and typically near or at the site of implantation of the mesh 250. Particularly, the handle 220 of the introducer 200 can be manipulated by medical personnel with or without an intermediate surgical tool, to pass the cape 240 and mesh 250 completely through the trocar 260 into the patient.

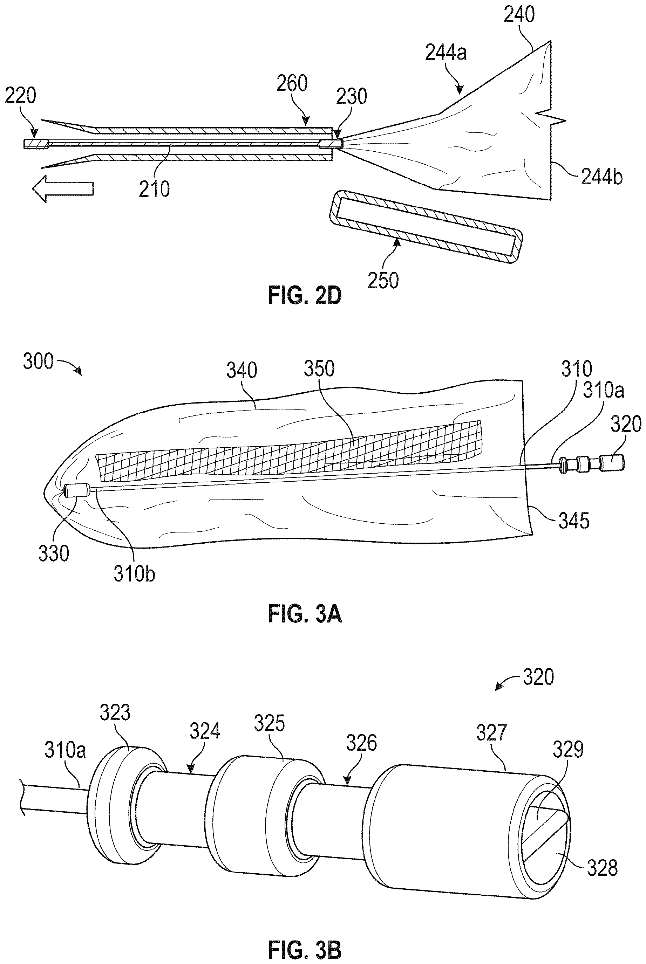

As depicted in Fig. 2D, once free of the restraints of the wall of the trocar 260, the cape 240 at least partially inverts unwrapping the mesh 250 and freeing the mesh 250 of the cape pocket 245. In some embodiments, at least a majority, if not an entirety, of the cape 240 inverts inside the patient. In some embodiments, the cape 240 automatically inverts upon exiting the trocar inside the patient, and particularly following a moving of the introducer 200 (and/or push stick 210) in a proximal direction, i.e., back through the trocar 260 and/or out of the patient.

As illustrated in Figs. 2A-2D, in some embodiments, the cape 240 is designed to transition between an expanded configuration (Figs. 2A-2B), an intermediate wrapped configuration (Fig. 2C), and a delivery configuration (Fig. 2D). In the expanded configuration, the cape can be at its widest or at least sufficiently wide to receive the mesh therein prior to wrapping. In the wrapped configuration, both the cape and the mesh are at their narrowest or at least sufficiently narrower than the expanded configuration to be passed through a trocar into a patient. In the delivery configuration, the cape may be sufficiently inverted and/or unwrapped to allow the mesh to be free of the introducer inside the patient.

As particular depicted in Fig. 2D, in the inverted delivery configuration, at least a portion, if not a majority or entirety, of an interior surface 244a of the cape 240 becomes an exterior surface of the cape 240 while the exterior surface 244b becomes an interior surface of the cape 240. In the inverted configuration, at least a portion, if not a majority or entirety, of the cape 240 extends distally from cape connector 230.

In some embodiments, the cape can be in an unwrapped configuration inside the patient by rotating, particularly via the handle, in a counterclockwise (or clockwise) direction to unwrap the cape around the mesh (and/or the stick). The unwrapped configuration occurs prior to inversion of the cape.

In some embodiments, the cape naturally transitions to an unwrapped configuration after exiting the trocar inside the patient thereby releasing the mesh.

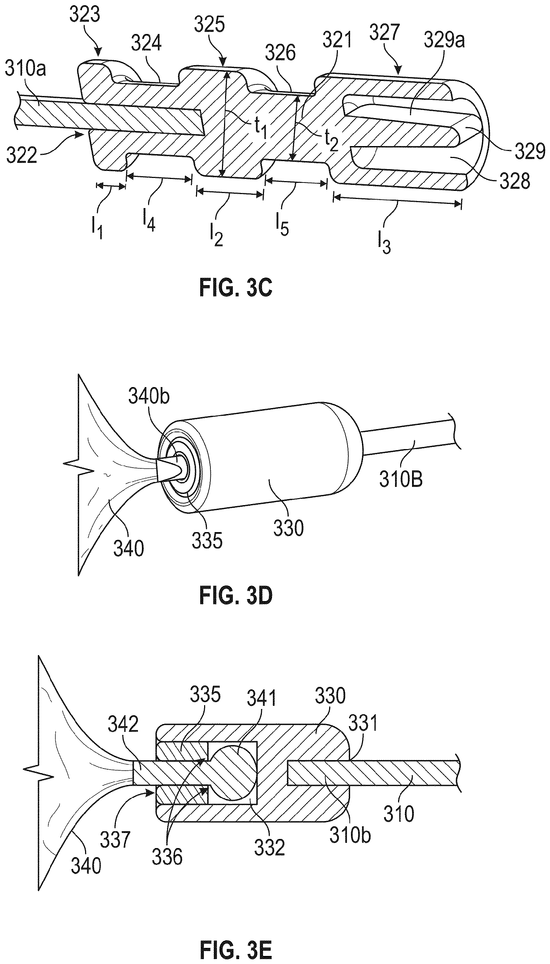

Turning to Figs. 3A-3E, an introducer 300 is depicted including a shaped handle 320, a shaped cape connector 330, and a removable (or interchangeable) cape 340. Particularly, Fig. 3A depicts an implantable surgical mesh introducer 300 including a removable cape 340 (in an expanded configuration) secured to a distal end portion 310b of a push stick 310 by the shaped cape connector 330. The shaped handle 320 is secured to a proximal end portion 310a of the push stick 310. An implantable surgical mesh 350 in a compact configuration, i.e., rolled, and/or folded, is located in a pocket 345 defined by the cape 340.

In some embodiments, the cape 340 can be transparent allowing the mesh 350 to be visually observed prior to, during, and/or after insertion into a patient. A transparent cape allows the medical personnel to easily determine if the mesh is damaged and/or contaminated in some manner prior to implantation and without having to remove the mesh from the cape.

Figs. 3B-3C depict the shaped handle 320 in more detail. The handle 320 may define a shaped body 321 including a distal lumen 322 configured to receive the proximal end portion 310a of the push stick 310 therein and a proximal lumen 328 including a grasping beveled bar 329 defined therein.

The beveled bar 329 is configured to be grasped by a surgical tool, such as a jaw member of surgical grasper. When the length of the push stick 310 is not sufficient to fully introduce the cape 340 and/or mesh 350 into a patient, the grasping beveled bar 329 can be grasped by a surgical tool, and particularly a pair of jaw members of a surgical grasper, to push the introducer 300 further into the patient. After completely introducing the cape 340 and/or mesh 350 into the patient, the mesh 350 is released of the introducer 300 and/or cape 340, and the introducer 300 (including the cape 340) can be easily removed from the patient via the same surgical tool still secured to the beveled bar 329. In addition, the grasping beveled bar 329 is helpful in rotating the push stick 310 during wrapping and/or unwrapping of the cape 340 around the mesh 350.

The proximal end portion 310a of the push stick 310 may be secured inside the distal lumen 322 of the handle 320 using any suitable manner. In some embodiments, the push stick 310 is glued to the handle 320. In some embodiments, the handle 320 is over-molded to the push stick 310. The handle 320 may alternatively be swaged, crimped, or screwed to the push stick 310 as well.

The shaped handle body 321 may include a plurality of handle rings 323-327 of alternating thickness (and/or circumference) t₁, t₂. For example, the thickness (and/or circumference) t₁ of the first, third, and fifth handle rings 323, 325, and 327, may generally be the same to each other but greater than the thickness t₂ (and/or circumference) of the second and fourth handle rings 324 and 326. While the thickness (and/or circumference) t₁ of the first, third, and fifth handle rings 323, 325, and 327 may be constant, in some embodiments, the longitudinal length l₁-l₃ of each of the first, third, and fifth handle rings 323, 325, and 327 may increase proximally (e.g., l₁≤ l₂≤l₃) while the longitudinal length l₄, l₅ of the second and fourth rings 324, 326 remain equal to each other (e.g., l₄=l₅). Although depicted as including five (5) rings, the handle 320 may include any number of handle rings of various lengths and/or thicknesses.

Figs. 3D-3E depict the shaped cape connector 330 designed to secure the distal end portion 340b of the cape 340 to the distal end portion 310b of the push stick 310. In some embodiments, the cape connector 330 includes a proximal opening 331 configured to receive the distal end portion 310b of the push stick 310 therein and a distal opening 332 configured to receive a bulbous part 341 of the distal end portion 340b of the cape 340 therein. Particularly, the distal end portion 340b of the cape 340 may be twisted, rolled, folded, and/or tied into a cape knot to form a bulbous cape part 341 prior to being inserted into the distal opening 332 of the connector 330.

As further depicted in Figs. 3D-3E, the connector 330 may include a crimping ring 335 configured to lock the bulbous cape part 341 of the cape 340 inside the distal opening 332 of the connector 330. The crimping ring 335 may include an aperture 337 configured to allow a cord 342 of the cape 340 extending from the bulbous part 341 to pass therethrough. The crimping ring 335 may include one or more locking tabs 336 configured to pinch into the cape cord 342 distal the bulbous cape part 341. The cape 340 transitions from the bulbous part 341 to the expanded cape 340 via the cape cord 342.

In some embodiments, one or more of the cape 340 or the crimping ring 335 is designed to be removable from the cape connector 330 to allow the cape 340 and/or the crimping ring 335 to be replaceable. In some embodiments, the introducers described herein are configured to be secured to any number of different interchangeable capes and/or crimping rings. For example, the capes and/or crimping rings may be made of different materials and/or made in different sizes to better accommodate the mesh to the introducer.

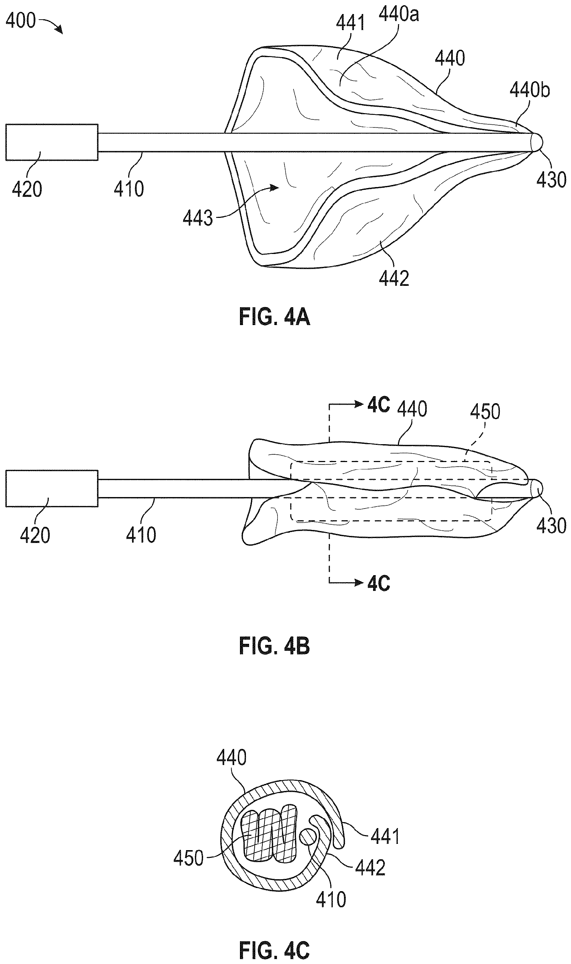

In some embodiments, as shown in Figs. 4A-4C, a mesh introducer 400 as described herein includes a cape 440 which does not completely surround a length of the push stick 410 and includes first and second side wings 441, 442 separated by a longitudinal gap 443. Unlike the cape 340 of Fig. 3A, the cape 440 does not form a cape pocket 445 only open on a proximal end 440a thereof. Rather, the cape 440 also includes a longitudinal gap 443 extending from the proximal end 440a of the cape 440 towards the distal end portion 440b of the cape 440 separating the first and second side wings 441, 442 from each other. By incorporating a longitudinal gap 443 of at least some length (if not a majority of the length or all of the length of the cape), the cape 440 including spaced apart wings 441, 442 is able to form an adjustable pocket configured to adapt to the size of the compacted mesh 450 and/or the design of the push stick 410.

Figs. 4B and 4C depict the introducer 400 in the wrapped configuration with the cape 440 and/or wings 441, 442 wrapped completely around the mesh 450 and at least a portion of the stick 410. The wings 441, 442 over-lapping each other in the wrapped configuration. It is envisioned that in some embodiments, the wings 441, 442 are configured to completely wrap only around the compact mesh while leaving the push stick uncovered by the cape.

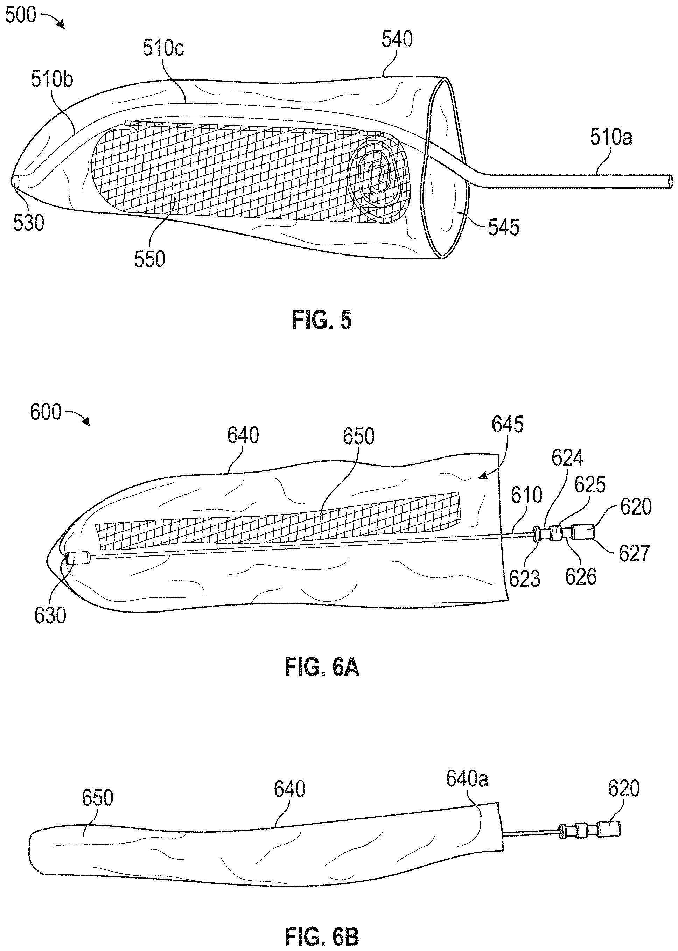

Fig. 5 depicts another introducer 500 wherein the push stick 510 includes a bend and/or curve 510c along a length of the stick 510, and particularly towards the distal end portion 510b of the stick 510. In some embodiments, the bend or curve 510c is designed to make room to one side of the stick 510 for the compacted mesh 550 to be positioned inside the pocket 545 of the cape 540. It is further envisioned that the bend or curve provides the stick with an enhanced strength or rigidity when exposed to stress and/or force during insertion into a patient.

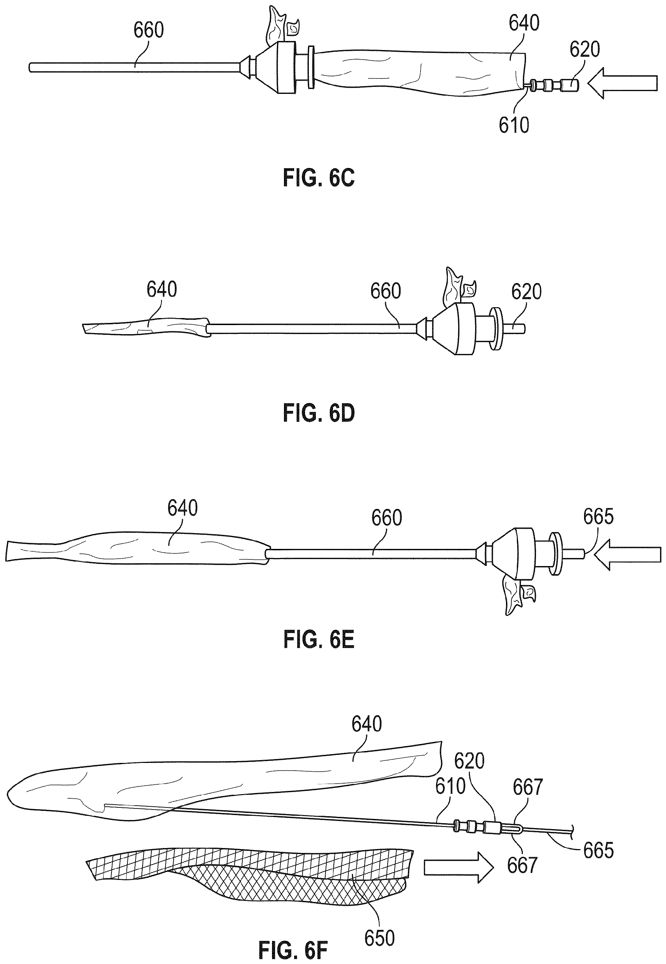

Figs. 6A-6F depict a method of using any of the mesh introducers described herein to deliver one or more implantable-mesh into a patient. Although the methods are directed specifically to the use of a trocar, the methods of delivery described herein may also be used in a generally similar manner without a trocar.

Initially, the implantable-mesh 650 can be folded and/or rolled into any compacted configuration prior to insertion into a pocket 645 defined by the cape 640. Since the cape 640 is flexible, medical personnel is afforded the opportunity to choose an ideal compact configuration of the mesh prior to being placed inside the cape. Any implantable-mesh can be used.

By implantable, the mesh described herein is configured to be positioned at a location within a body for any sufficient amount of time to at least temporarily treat and/or repair a soft tissue defect, such as a hernia or prolapse.

In the context of this application the term "mesh," "surgical mesh," or "implantable-mesh" refers to an arrangement of biocompatible filaments or yarns (e.g., a knitted fabric, a woven fabric, or a nonwoven fabric) arranged in a manner to include pores within the mesh face that can encourage tissue ingrowth. The mesh can be bioresorbable, partially bioresorbable or non-bioresorbable. The mesh includes first and second opposite faces and an outer perimeter which defines a center of the mesh on each face. The mesh can also be provided in any shape (rectangular, square, circular, oval, etc.) and size. Such meshes are well known to the person skilled in the art.

In some embodiments, the implantable-mesh is a two-dimensional knitted fabric. In some embodiments, the implantable-mesh is a three-dimensional knitted fabric. In some embodiments, the implantable-mesh is a self-fixating mesh (e.g., ProGrip^{®} mesh by Medtronic). In some embodiments, the mesh includes one or more layers of fabric and may optionally include an anti-adhesion barrier layer positioned on at least one portion or one side of the fabric thereby forming a composite mesh.

As illustrated in Fig. 6A, after the mesh 650 is compacted, the mesh 650 can be placed inside a pocket 645 defined by the cape 640 in an expanded configuration. The cape 640 completely surrounds a longitudinal length of the mesh 650. The cape 640 can also completely surround a longitudinal length of the push stick 610.

Next, as depicted in Fig. 6B, the cape 640 can transition from the expanded configuration loosely surrounding the mesh (Fig. 6A) to a wrapped configuration tightly wrapped around the mesh. In the wrapped configuration, the outer diameter (and/or perimeter) of the cape 640 is greatly reduced around the mesh 650.

The overall volume of the introducer and the mesh in the wrapped configuration is lower as compared to the cape free combination of a mesh and 5 mm arm of a standard surgical grasper of Figs. 1A-1D. The overall force needed to introduce the mesh through the trocar is also lower due to the reduced overall volume of the caped introducer/mesh combinations described herein. The overall introducing force may also be lower due to low coefficient of friction of the cape material as compared to the mesh alone. Any stresses and/or forces are applied directly to the cape and/or the push stick, but not directly to the mesh.

In addition, by surrounding the mesh, the cape prevents the mesh from contamination via direct contact prior to and/or during insertion into the patient. For example, a surgical mesh can be compacted and wrapped in the cape by any medical personnel prior to surgery and left on a sterile surface or table during the surgery. In addition, by surrounding the mesh, the cape generally prevents the mesh from any direct contact until needed. In the case of robot assisted surgical procedures, it is envisioned that medical personnel inside the sterile surgical area may be able to place and/or wrap the mesh inside the cape prior to implantation, while the surgeon remains in a non-sterile robotic console.

Also, by holding the mesh inside the cape with the mesh unattached to the introducer, the mesh is never pinched, grasped, or put under any direct stress by any portion of the introducers described herein, i.e., the cape, the push stick, the cape connector and/or the handle. Thus the mesh introducers described herein do not cause any physical damage, i.e., cutting, stretching, denting, etc. to the mesh during insertion.

Although the least amount of stress and/force may be applied to the mesh when passed through a trocar in the wrapped configuration, in some embodiments, the mesh may be passed through a trocar inside a cape in the expanded configuration wherein the inner walls of the trocar force the flexible cape to narrow around the mesh as the introducer is passed through the trocar.

As further depicted in Figs. 6A and/or 6B, the free proximal end portion 640a of the cape 640, in either pre-insertion configurations, may not typically extend to and/or beyond the handle 620 of the push stick 610 to allow for maximum exposure of the handle 620 during insertion.

As shown in Figs. 6C-6F, the caped mesh introducer 600 can be inserted (e.g., pushed, slid, or moved distally) through a trocar 660 into a patient until the cape 640 and the mesh 650 are completely inserted through the trocar 660 and inside the patient.

As particularly shown in Fig. 6C, the distal end portion of the push stick 610 (and/or cape 640) in the wrapped configuration is introduced into the trocar 660.

Next, as depicted in Fig. 6D, the introducer 600 including the mesh 650 can be inserted through the trocar 660 via the handle 620, either manually by medical personnel (directly or via an intermediate handheld surgical tool). In some embodiments, as further depicted in Fig. 6D, the push stick is not long enough to pass the cape 640 (and/or mesh) completely through the trocar 660 while the handle 620 remains outside the trocar 660. For example, in some embodiments, medical personnel can directly push the handle of the introducer into the trocar until the handle is generally the only portion of the introducer sticking out of the trocar. Then, as depicted in Figs. 6E-6F, the handle 620 is grasped with a surgical tool 665, such as a surgical grasper, and inserted (e.g., pushed, slid, or moved distally) further through the trocar 660 until the introducer 600 including the cape 640 and the mesh 650 are completely inside the patient. In particular embodiments, the jaw members 667 of the surgical grasper 665 pinch an exterior of the handle 620, such as any one or more of the rings 623-627 of the handle 620. Alternatively, in some embodiments, the jaw members 667 of the surgical grasper 665 are of sufficient size to pinch a grasping beveled bar (best seen in Fig. 3D-3E) positioned within a proximal lumen of the handle 620.

Once the cape 640 and mesh 650 are completely inside the patient, at least a portion, if not all, of the cape 640 can open (automatically or via rotating of the introducer) releasing the compacted mesh 650 from the introducer 600 and free into the patient. In some embodiments, at least a majority of the cape 640 transitions to an inverted delivery configuration (schematically depicted in Fig. 2D) freeing the mesh 650 of the cape 640. The introducer 600 can be withdrawn from the patient through the trocar 660 by moving the introducer 600 in a proximal direction out of the trocar 660 leaving while the mesh 650 in the patient.

In some embodiments, wherein the cape includes a plurality of wings separated by a longitudinal gap, the wings automatically separate along the longitudinal gap after being free of the restraints of the trocar to release the mesh into the patient.

The free compacted mesh inside the patient may be unrolled and/or unfolded. In some embodiments, the mesh maintains a stiffness and/or memory to naturally unroll and/or unfold inside the patient. In some embodiments, the mesh has to be physically unrolled and/or unfolded by the surgeon laparoscopically or via robotic assistance.

Methods of repairing and/or treating of a soft tissue defect are also described herein. For example, a method of treating and/or repairing a soft tissue defect, such as a hernial defect or tissue prolapse, includes the steps of: inserting a mesh in a compacted configuration into a cavity of a patient via a mesh introducer including a cape surrounding the mesh (e.g., the cape in an expanded or wrapped configuration); opening of the cape (e.g., transitioning the cape into an inverted and/or open delivery configuration) inside the patient freeing the mesh of the introducer; deploying the mesh into an unrolled configuration; positioning and fixating the mesh in or around the soft tissue defect; and withdrawing the mesh introducer including the cape from the abdominal cavity of the patient. In some embodiments, surgical trocars are used to pass the mesh and/or introducer into and out of the patient.

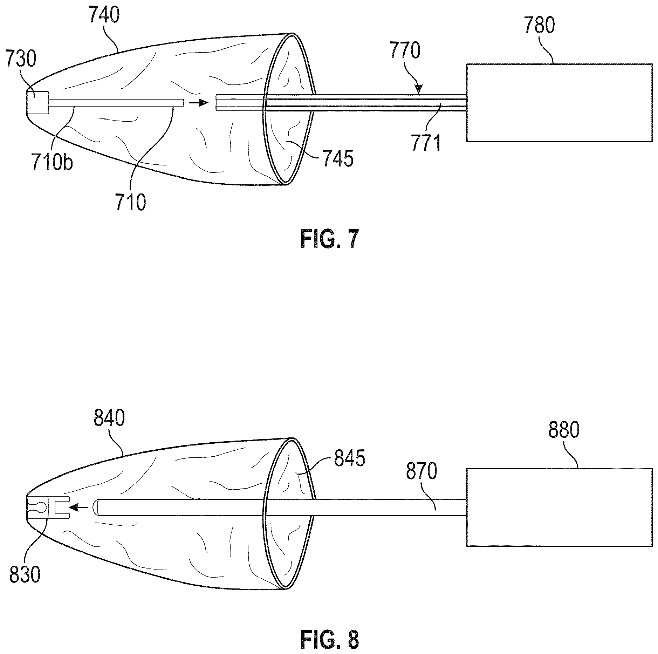

Turning to Figs. 7 and 8, some additional alternative introducers 700, 800 are described. In Fig. 7, the mesh introducer 700 is configured to be added to a separate surgical arm 770 of either a surgical tool or robotic surgical system, as represented by box 780. The introducer 700 includes a cape 740 secured to a distal end portion 710b of a push stick 710 via a cape connector 730 wherein the proximal end portion of the stick 710 is designed to be received in and/or secured within a hollow portion 771 of a surgical arm 770. In some embodiments, the surgical arm 770 is part of surgical tool 780. In some embodiments, the surgical arm 770 is part of a robot and/or a robotic surgical system 780.

The cape 740 and the push stick 710 of Fig. 7 are designed to be removable from the surgical arm 770 after the mesh is introduced into the patient. It is in envisioned that following the introduction of the mesh into the patient, the cape and push stick can be removed from the surgical arm and replaced with an end effector suitable for some other surgical function, such as grasping, cutting, stapling, tacking, suturing, lighting, sensing, videoing, etc.

In Fig. 8, the mesh introducer 800 only includes a cape connector 830 secured only to a distal end portion 840b of a cape 840. The distal end portion 830b of the connector 830 is designed to secure the cape 840 thereto and the proximal end portion 830a of the connector 830 is designed to receive and/or secure a surgical arm 870 therein (as indicated by the arrow). In some embodiments, the surgical arm 870 is part of surgical tool 880. In some embodiments, the surgical arm 870 is part of a robot and/or a robotic surgical system 880.

The connector (or the stick) and the arm may be secured to each other using any suitable manner. In some embodiments, the connector (or the stick) and the arm are configured to snap together. In some embodiments, at least one of the connector (or the stick) and/or the arm are threaded to screw the two components together. In some embodiments, the connector (or the stick) and the arm are complimentary male/female parts configured to frictional fit one within the other.

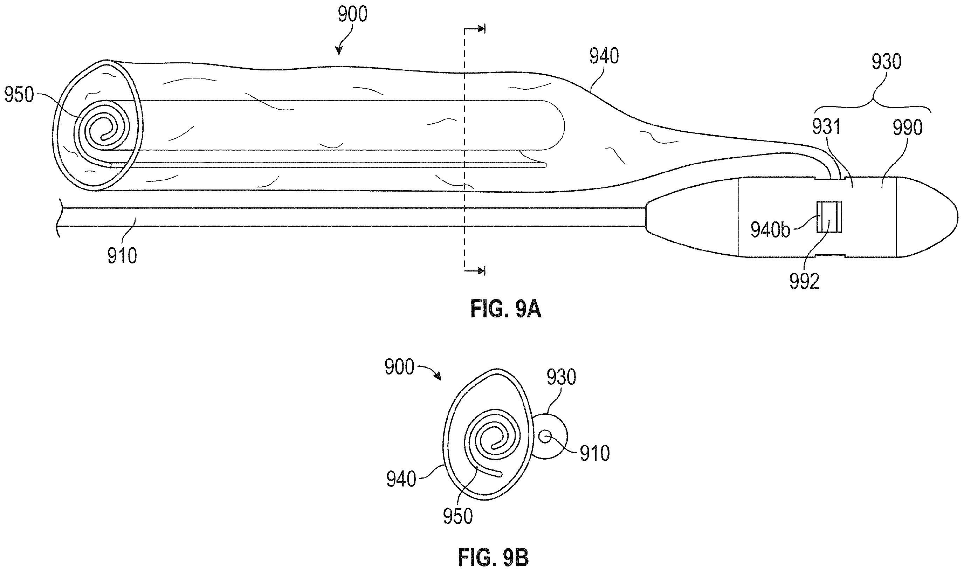

Figs. 9A-9B depict yet another mesh introducer 900 as described herein. The mesh introducer 900 includes a cape 940 which is configured to wrap around a compacted mesh 950 but not the push stick 910. The cape 940 as shown forms a cape pocket 945 open on a proximal end 940a thereof and free of the push stick 910 in both a wrapped (shown in Fig. 9A) and non-wrapped configuration (not shown in Fig. 9A) about the mesh 950. The cape 940, in some embodiments, further includes a longitudinal gap.

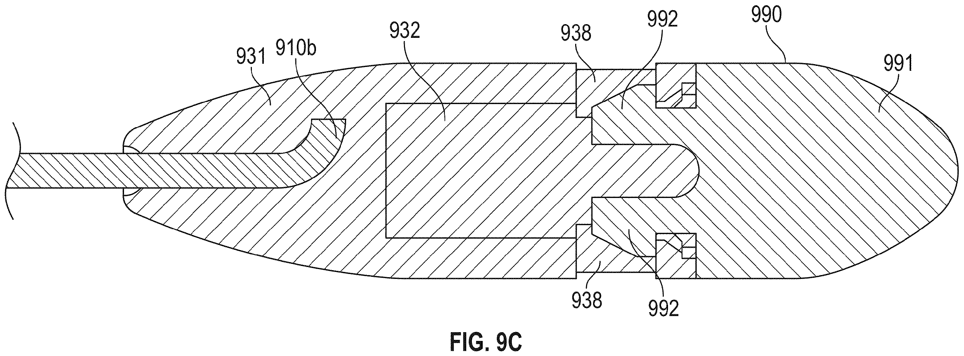

As shown in Figs. 9A and 9C, in some embodiments, the introducer 900 includes a two-piece cape connector 930 including a base part 931 and a cap part 990. The base part 931 is attached to a distal end portion 910b (particularly a curved distal end portion as shown) of the push stick 910. The base part 931 includes an inner cavity 932 configured to receive an end portion 940b of the cape 940 therein, one or more base locking recesses 938 defined therein and/or therethrough, and a base opening 939 defined therethrough for allowing the cape 940 to exit the base part 931. The cap part 990 includes a cap body 991 and one or more cap locking tabs 992 extending proximally therefrom. The one or more cap locking tabs 992 being configured to matingly engage the one or more base locking recesses 938 to form the cape connector 930. In some embodiments, the base and the cap parts are configured to snap together. In some embodiments, the base locking recesses completely pass through the base part making at least a portion of the locking tabs accessible from an exterior of the connector for easy removal (i.e., pinching of the locking tabs inside the locking recesses).

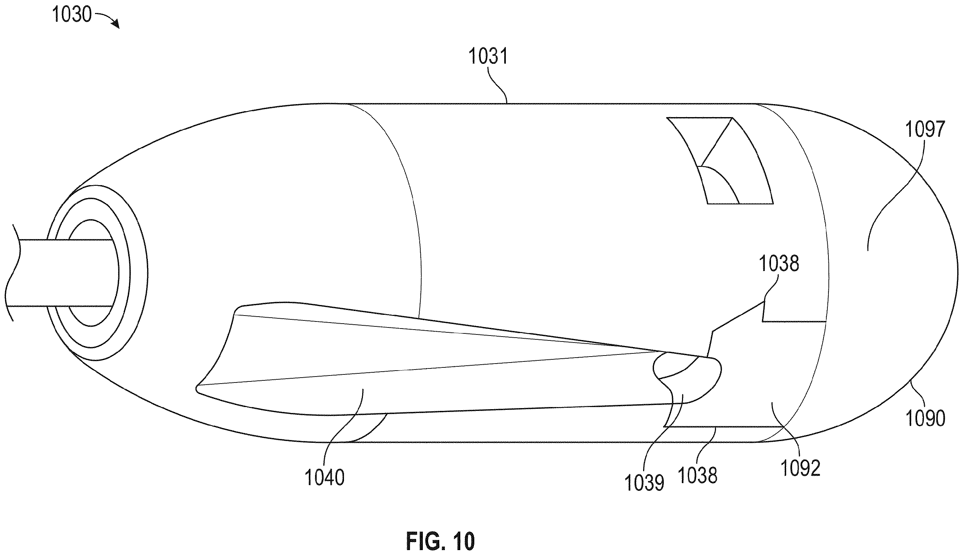

Additional multi-part cape connectors are depicted in Figs. 10-13. In some embodiments, as shown in Fig. 10, a base 1031 of a multi-part cape connector 1030 includes one or more base locking recesses 1038, one or more base openings 1039, and an exterior base channel 1037 configured to guide the cape 1040 proximally after exiting the base opening 1039. The cap part 1090 includes a cap body 1091 and one or more cap locking tabs 1092 extending proximally therefrom.

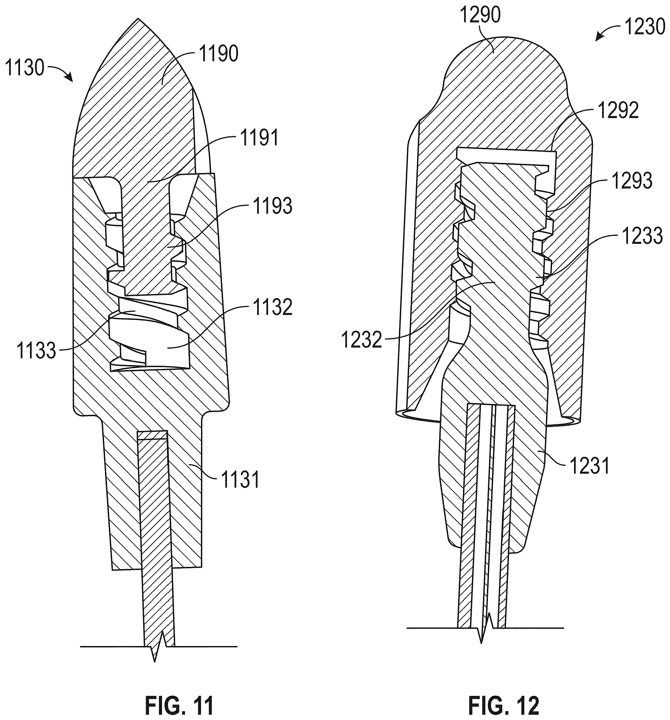

In some embodiments, as shown in Figs. 11 and 12, a multi-piece cape connector 1130, 1230 including a male/female connector base 1131, 1231 and cap 1190, 1290. In Fig. 11, the multi-piece cape connector 1130 includes a base part 1131 with an inner cavity 1132 defined therein and a cap part 1190 with a post 1191 extending proximally therefrom. The inner cavity 1132 includes an interior thread and/or notches 1133 and the post 1191 includes an exterior thread and/or notches 1193. In some embodiments, the interior and exterior threads are configured to interact with each other to screw the cap part, via the post, to the base part, via the base cavity. In some embodiments, the inner and outer notches are configured to interact with each other to lock the cap part and the base part together by simply being pressed together (i.e., without rotation) by inserting the post into the cavity.

In Fig. 12, the multi-piece cape connector 1230 includes a cap part 1290 with an inner cavity 1292 defined therein and a base part 1231 with a post 1232 extending distally therefrom. The inner cavity 1292 includes an interior thread and/or notches 1293 and the post 1232 includes an exterior thread and/or notches 1233. In some embodiments, the interior and exterior threads are configured to interact with each other to screw the cap part, via the cavity, to the base part, via the post. In some embodiments, the inner and outer notches are configured to interact with each other to lock the cap part and the base part together by simply being pressed together (i.e., without rotation) by inserting the post into the cavity.

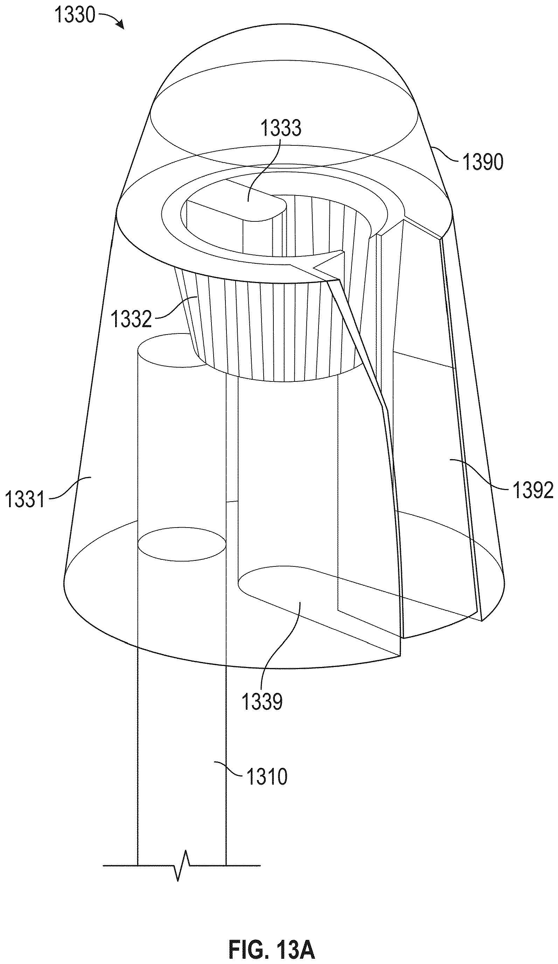

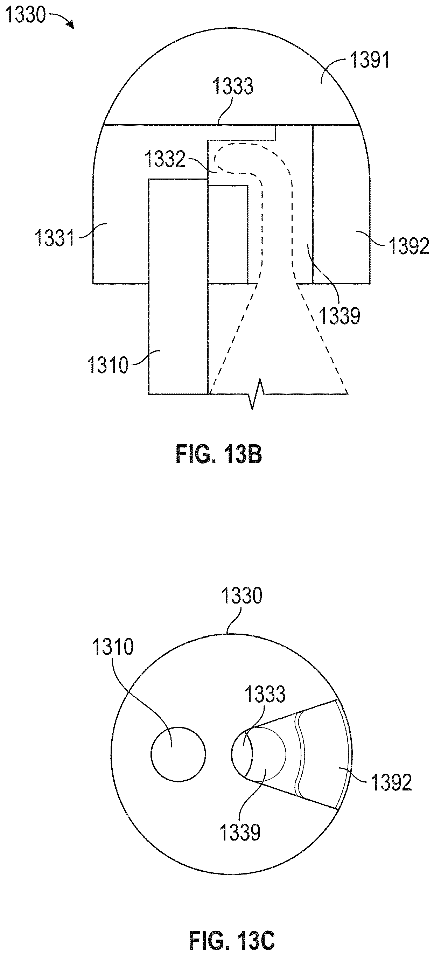

In some embodiments, as shown in Figs. 13A-13C, a multipiece cape connector 1330 includes a connector base part 1331 including an inner cavity 1332 positioned above, and in communication with, a base opening 1339. The inner cavity 1332 being configured to receive a distal end of a cape (shown in phantom in Fig. 13B) therein. An inner tab 1333 is positioned and/or extends over a portion, if not a majority, of the inner cavity 1332 to aid in holding the distal end portion of the cape in the inner cavity 1332. The base opening 1339 configured to allow the cape to pass therethrough to exit the connector 1330.

The connector 1330 also includes a connector cap part 1390 configured to matingly engage the base part 1331. The cap 1390 includes an exterior tab 1392 extending proximally from a body 1391 of the cap 1390. The exterior tab 1392 configured to guide the cape through the base opening 1339 by filling an outer portion of the base opening 1339.

As further shown in Figs. 13A-13C, in some embodiments, the introducers described herein include a push stick 1310 which is off-set and/or not centered on the cape connector 1330.

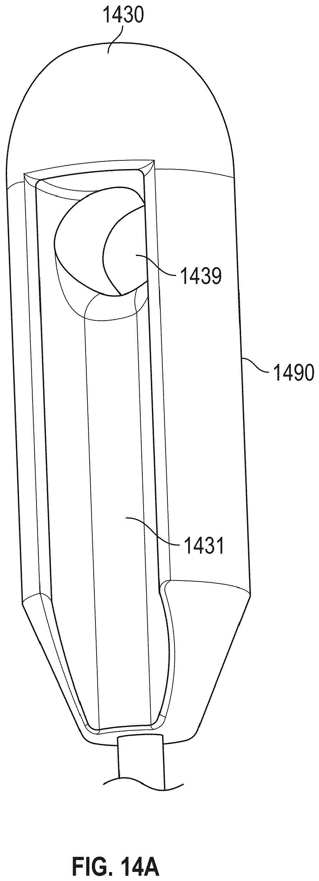

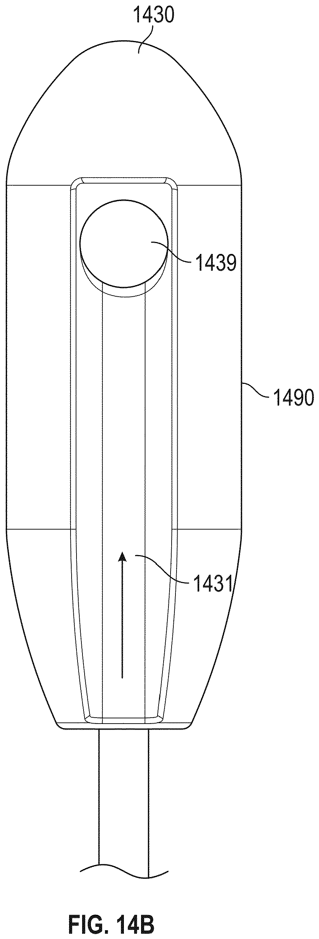

In some embodiments, as shown in Figs. 14A-14B, the cape connector is a single piece or monobloc cape connector 1430. The connector 1430 includes an opening 1439 defined therethrough configured to receive a distal end portion of a cape therein and/or therethrough. In some embodiments, as shown in Fig. 14B, an interior portion 1431 and an exterior portion 1490 of connector 1430 are configured to slide relative to each other thereby closing the opening 1439 and pinching the end portion of the cape therein. For example, as indicated by the arrow in Fig. 14B, the interior portion 1431 can be slid distally to reduce the size of the opening 1439.

Alternatively, a monobloc cape connector may not include slidable portions and the cape may be secured in the opening using any other suitable method, including but not limited to, tying, glue, adhesive, welding, and the like.

The cape connector can be made using any suitable method including but not limited to 3-D printing, injection molding, extrusion, pressing, and the like. The cape connector can also be made from any suitable biocompatible material including but not limited to polymeric materials such as high-density polyethylene, ultra-high molecular weight polyethylene, polyether ether ketone (PEEK), polyamides, polytetrafluoroethylene (PTFE), polyethylene terephthalate (PET), polypropylene, polycarbonate, acrylonitrile butadiene styrene (ABS), and/or metallic materials such as stainless steel, silver, gold, titanium, aluminum, and the like. The cape connector can be attached to the push stick using any suitable method including but not limited to over-molding, gluing, extruding, welding, crimping, threading, snap-fitting, and the like.

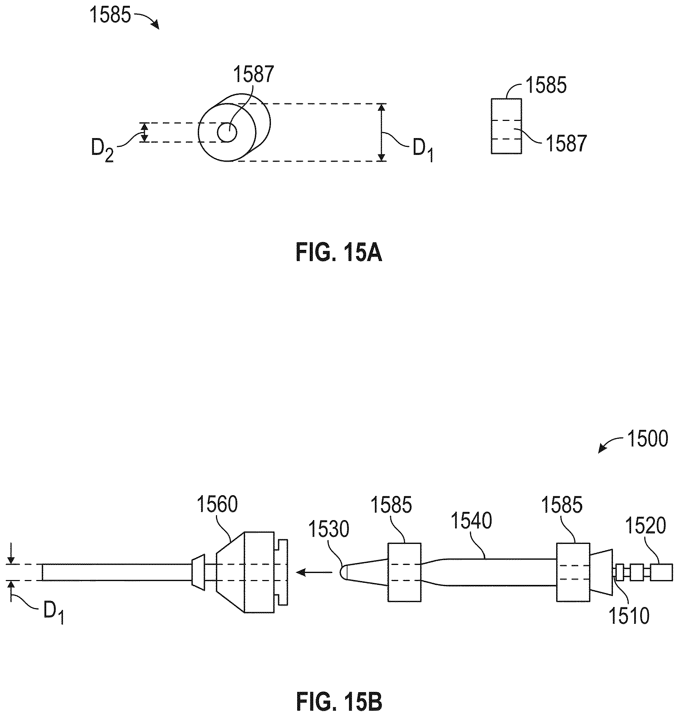

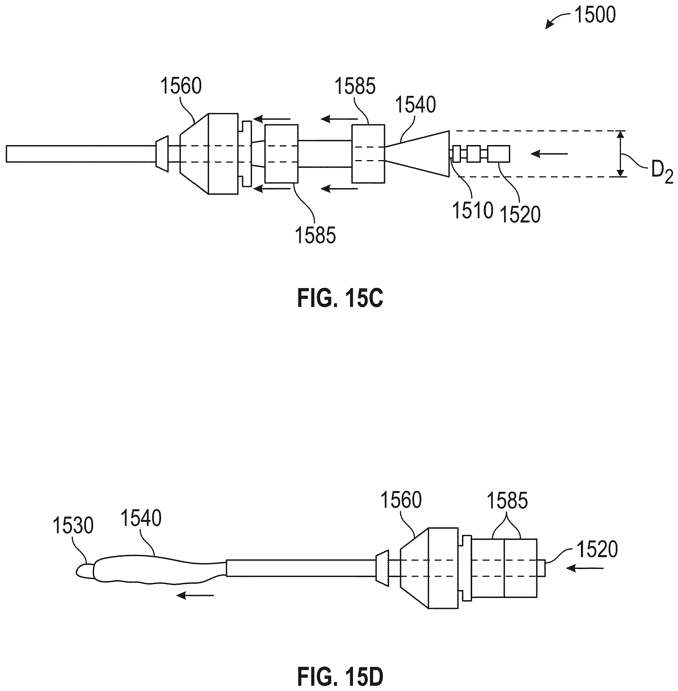

As further shown in Figs. 15A-15D, in some embodiments, a mesh introducer 1500 including a push stick 1510, handle 1520, cape connector 1530, and cape 1540 also further includes one or more mesh retainer rings 1585. As shown in Fig. 15A, the mesh retainer ring 1585 includes a generally centered retainer opening 1587 defined therethrough. The retainer ring 1585 defines an external diameter Di larger than a diameter di of the trocar 1560. The retainer opening 1587 defines an internal diameter D₂ of a size generally equal to or less than the diameter di of the trocar 1560 (and/or generally less than a diameter d₂ of the cape introducer 1500 in a wrapped configuration with a mesh therein). The retainer rings 1585 are configured to slide along the cape 1540 during the introduction of the cape 1540 (and/or mesh) through the trocar 1560, as indicated by the arrows, and remain outside the trocar 1560, as shown in Figs. 15B-15D.

In some embodiments, the cape system and/or introducer 1500 of Fig. 15B, can be prepared by a surgeon, or other medical personnel, prior to dissection and left on a sterile surface during dissection. The retainer rings 1585 supporting the introducer 1500 off the surface.

In the case of robotic surgery, in some embodiments, the surgeon works from a non-sterile environment and/or on a non-sterile robotic console during and/or after dissection without having to enter the sterile operating area to pass the introducer into the patient. Rather, other medical personnel positioned within the sterile operating area can introduce the cape system 1500.

Surgical kits including any of the mesh introducers described herein are also provided. A surgical kit includes at least one implantable-mesh introducer including a cape and at least one of a push stick and/or cape connector. The introducer may further include a handle and/or one or more retainer rings.

The surgical kits described herein may further include at least one or more of an implantable-mesh, a surgical tool, and/or a surgical trocar.

In some embodiments, the surgical kits described herein include at least an introducer and a plurality of different interchangeable capes and/or crimping rings.

In some embodiments, the surgical kits described herein include at least an introducer and a plurality of different interchangeable cape connectors including one-piece and multi-piece connectors.

In some embodiments, the surgical kits include an introducer, a first cape configured to completely surround a longitudinal length of the introducer and a second cape including a pair of side wings separated by a longitudinal gap. The first and second capes are configured to be removably secured to the introducer. The first and second capes are configured to be interchangeable with the introducer.

It will be understood that various modifications may be made to the embodiments disclosed herein. Thus, those skilled in the art will envision other modifications within the scope and spirit of the disclosure.

### Example 1

An implantable-mesh introducer was formed including a cape and a push stick. The cape was made of polyethylene high density film (260 mm by 100 mm) having a thickness of 10 micrometers. The push stick was made of stainless steel having a diameter of 1.6 mm and a length of 280 mm.

A cape connector was glued to a distal end portion of the push stick and a handle was glued to a proximal end portion of the stick. The cape connector (including a crimp ring) and the handle were made of acrylonitrile butadiene styrene (ABS) and formed via 3D-printing. The cape connector defined a cylindrical shape having a 7.2 mm diameter and 15.5 mm length. The crimp ring was also cylindrical having a 4.6 mm diameter and 4 mm length. The crimp ring fits into a distal opening of the connector. The handle defined several rings of varying diameters and length to improve gripping of the handle. The largest diameter of the handle was 8 mm and the length was 33 mm. The proximal end portion of the handle included a grasping beveled bar located within a proximal lumen of the handle.

The cape was secured to the push stick by the cape connector including the crimp ring. A distal end portion of the cape was tied into a knot. The cape knot was located within the distal opening of the cape connector with a cape collar extending therefrom. The crimp ring was positioned around the cape collar and secured there to by crimping tabs of the crimping ring. The crimping ring being positioned within the distal opening of the connector and distal the cape knot.

## Claims

1. An implantable-mesh introducer comprising:
a push stick extending between a proximal end portion and a distal end portion; and
a cape extending between a free proximal end portion and a fixed distal end portion, the fixed distal end portion of the cape secured to the distal end portion of the push stick.

2. The implantable-mesh introducer of claim 1, wherein the cape is formed from a sheet of high-density polyethylene.

3. The implantable-mesh introducer of claim 2, wherein the cape comprises a thickness less than about 100 microns.

4. The implantable-mesh introducer of claim 1, wherein the push stick comprises a stainless steel push stick.

5. The implantable-mesh introducer of claim 1, wherein the push stick comprises a handle on the proximal end portion of the push stick.

6. The implantable-mesh introducer of claim 5, wherein the handle comprises a distal lumen configured to receive the proximal end portion of the push stick and a proximal lumen including a beveled grasping bar located within the proximal lumen.

7. The implantable-mesh introducer of claim 6, wherein the handle further comprises a shaped body defined by a plurality of first rings each having a first thickness separated by one or more second rings each having a second thickness, the first thickness being greater than the second thickness.

8. The implantable-mesh introducer of claim 7, wherein a length of each first ring of the plurality of first rings increases in a proximal direction while a length of each of the one or more second rings remains the same.

9. The implantable-mesh introducer of claim 1, wherein the distal end portion of the push stick comprises a cape connector configured to secure the fixed distal end portion of the cape to the distal end portion of the push stick.

10. The implantable-mesh introducer of claim 9, wherein the cape connector comprises a distal lumen defined therein and the distal lumen of the cape connector is configured to receive the fixed distal end a portion of the cape.

11. The implantable-mesh introducer of claim 10, wherein the fixed distal end portion of the cape includes a bulbous part positioned within the distal lumen of the cape connector and a cape cord extending from the bulbous part and out of the distal lumen of the cape connector.

12. The implantable-mesh introducer of claim 11, wherein the cape connector further comprises a crimping ring configured to snap into the distal lumen of the connector distal of the bulbous part of the cape to maintain the bulbous part of the cape within the distal lumen of the cape connector.

13. The implantable-mesh introducer of claim 12, wherein the crimp ring surrounds the cape cord inside the distal lumen of the cape connector and includes one or more locking tabs configured to pinch the cape cord inside the distal lumen of the cape connector.

14. The implantable-mesh introducer of claim 9, wherein:
the cape connector is a multi-piece cape connector including a base part and a cap part;
the base part is attached to the distal end portion of the push stick;
the base part includes an inner cavity configured to receive an end portion of the cape, one or more base locking recesses, and a base opening defined through the base part to allow the cape to exit the cape connector; and
the cap part includes a cap body and one or more cap locking tabs configured to matingly engage the one or more base locking recesses to form the cape connector.

15. The implantable-mesh introducer of claim 14, wherein the cape connector further comprises an exterior base channel defined in an exterior of the base part, the exterior base channel is configured to guide the cape proximally after exiting the base opening.

16. The implantable-mesh introducer of claim 9, wherein:
the cape connector is a multi-piece cape connector including a base part and a cap part;
the base part is attached to the distal end portion of the push stick;
the base part includes an inner cavity configured to receive an end portion of the cape, the inner cavity including an interior thread or notch; and
the cap part includes a post with an exterior thread or notch, wherein the interior and exterior threads or notches are configured to interact with each other to attach the cap part to the base part.

17. The implantable-mesh introducer of claim 9, wherein:
the cape connector is a multi-piece cape connector including a base part and a cap part;
the base part is attached to the distal end portion of the push stick;
the base part including a post including an exterior thread or notch; and
the cape part includes an inner cavity configured to receive an end portion of the cape, the inner cavity including an interior thread or notch, wherein the interior and exterior threads or notches are configured to interact with each other to attach the cap part to the base part.

18. The implantable-mesh introducer of claim 9, wherein the cape connector is a one-piece cape connector including a single opening configured to receive a distal end portion of a cape through the cape connector.

19. The implantable-mesh introducer of claim 18, wherein the cape connector is configured to pinch the distal end portion of the cape within the opening.

20. The implantable-mesh introducer of claim 1, further comprising at least one mesh retainer ring configured to slide along an exterior of the cape during introduction of the cape or mesh through the trocar, wherein a generally centered retainer opening is defined through the at least one mesh retainer ring.

21. The implantable-mesh introducer of claim 20, wherein:
the at least one mesh retainer ring is configured to remain outside the trocar;
the at least one mesh retainer ring defines an external diameter larger than a diameter of the trocar; and
the retainer opening defines an internal diameter generally equal to or less than the diameter of the trocar.

22. A method of delivering an implantable-mesh into a patient comprising
placing a compacted implantable-mesh inside a cape of an implantable-mesh introducer;
pushing the implantable-mesh introducer including the compacted implantable-mesh placed inside the cape through a trocar into a patient until the cape and the mesh are completely inserted through the trocar; and
releasing the compacted implantable-mesh from inside the cape into the patient.

23. The method of claim 22, wherein placing the compacted implantable-mesh comprises placing the compacted implantable-mesh into a pocket defined by the cape in an expanded configuration, the compacted implantable-mesh being positioned between a push stick of the introducer and an inner surface of the cape.

24. The method of claim 23, further comprising rotating the implantable-mesh introducer transitioning the cape from an expanded configuration to a wrapped configuration wherein the cape is wrapped closely around the compacted mesh.

25. The method of claim 22, wherein pushing the implantable-mesh introducer through a trocar initially is performed by hand until only the handle of the introducer remains outside the trocar and then a surgical tool can be attached to the handle to further push the entire implantable-mesh introducer through the trocar and into the patient.

26. The method of claim 22, wherein releasing the compacted mesh further comprises the cape transitions to a delivery configuration wherein the cape automatically opens or inverts inside the patient and outside the trocar to free the compacted mesh from inside the cape pocket.

27. A method of repairing a soft tissue defect comprising:
inserting a mesh in a compacted configuration into a cavity of a patient via a mesh introducer including a cape surrounding the mesh;
opening of the cape inside the patient freeing the mesh of the introducer;
deploying the mesh into a non-compacted configuration;
fixating the mesh in or around the soft tissue defect; and
withdrawing the mesh introducer including the cape from the cavity of the patient.

28. A surgical kit comprising:
an implantable-mesh introducer including a cape and at least one of a push stick or a cape connector; and
at least one of an implantable-mesh, a surgical grasper, or a trocar.
